## Europäisches Patentamt

## European Patent Office

## Office européen des brevets

(19)

(11) Veröffentlichungsnummer: **0 109 129**
**B1**

(12) **EUROPÄISCHE PATENTSCHRIFT**

(45) Veröffentlichungstag der Patentschrift:
**28.01.87**

(51) Int. Cl.⁴: **G 01 S 7/52,** G 01 S 15/89,
A 61 B 10/00

(21) Anmeldenummer: **83201606.7**

(22) Anmeldetag: **10.11.83**

(54) Verfahren und Anordnung zur Laufzeitbestimmung eines Ultraschallimpulses.

(30) Priorität: **16.11.82 DE 3242284**

(43) Veröffentlichungstag der Anmeldung:
**23.05.84 Patentblatt 84/21**

(45) Bekanntmachung des Hinweises auf die Patenterteilung:
**28.01.87 Patentblatt 87/5**

(84) Benannte Vertragsstaaten:
**BE DE FR GB SE**

(56) Entgegenhaltungen:
DE - A - 2 737 109
DE - A - 2 827 423
DE - B - 2 919 381
US - A - 4 317 369

(73) Patentinhaber: **Philips Patentverwaltung GmbH,
Billstrasse 80, D-2000 Hamburg 28 (DE)**

(84) Benannte Vertragsstaaten: **DE**

(73) Patentinhaber: **N.V. Philips' Gloeilampenfabrieken,
Groenewoudseweg 1, NL-5621 BA Eindhoven (NL)**

(84) Benannte Vertragsstaaten: **BE FR GB SE**

(72) Erfinder: **Schomberg, Hermann, Dr.,
König-Heinrich-Weg 127d, D-2000 Hamburg 61 (DE)**

(74) Vertreter: **Hartmann, Heinrich, Dipl.-Ing. et al, Philips
Patentverwaltung GmbH
Billstrasse 80 Postfach 10 51 49,
D-2000 Hamburg 28 (DE)**

# Beschreibung

Die Erfindung bezieht sich auf ein Verfahren zur Laufzeitbestimmung eines Ultraschallimpulses, der einen Untersuchungsbereich durchsetzt und in ein elektrisches Messignal umgesetzt wird, wobei der Zeitraum gemessen wird, der nach dem Aussenden des Ultraschallimpulses verstreicht, bis das Messignal einen vorgegebenen Wert erreicht hat.

Ein solches Verfahren wird z.B. bei der Ultraschall-Computertomographie benötigt, um die Schallbrechungsindexverteilung bzw. die Geschwindigkeitsverteilung im Untersuchungsbereich anhand der Laufzeiten zeitlich aufeinanderfolgender Ultraschallimpulse zu rekonstruieren.

Verfahren und Anordnungen dieser Art sind bekannt aus der DE-OS 27 37 109 (US-PS 4 075 883) sowie aus der DE-OS 28 27 423 (US-PS 4 279 157). Die bekannten Verfahren und Anordnungen liefern ein Bild der Brechungsindexverteilung bzw. der Geschwindigkeitsverteilung in einer Schicht eines zwischen einer Ultraschallsende- und einer Ultraschallempfängeranordnung befindlichen Objektes. Eine Rekonstruktionseinheit bestimmt für eine Vielzahl von Punkten dieser Schicht jene Parameter aufgrund der gemessenen Laufzeiten der Ultraschallimpulse vom Sender zum Empfänger. Damit die rekonstruierte Verteilung mit der tatsächlichen möglichst genau übereinstimmt, muss u.a. die Laufzeit der Ultraschallimpulse sehr genau bestimmt werden, z.B. aus 20 ns genau.

Zur Bestimmung der Laufzeit wird bei den bekannten Anordnungen der Zeitraum zwischen dem Aussenden eines Ultraschallimpulses und dem Zeitpunkt bestimmt, in dem das von der Empfängeranordnung empfangene Signal einen vorgebbaren Schwellenwert überschreitet, der im allgemeinen unmittelbar oberhalb des Störpegels liegt. Die auf diese Weise bestimmte Laufzeit ist genügend genau, wenn bei den verschiedenen Messungen die Signale wenigstens annähernd die gleiche Amplitude haben und wenn die Störungen z.B. durch Rauschen verhältnismässig klein sind. In der Praxis sind diese Voraussetzungen jedoch meist nicht erfüllt, wodurch sich grössere Messfehler ergeben können als zulässig.

Aufgabe der vorliegenden Erfindung ist es, die Laufzeit mit der erforderlichen Genauigkeit zu bestimmen.

Diese Aufgabe wird erfindungsgemäss dadurch gelöst,
- dass das Messignal mit einer vorgegebenen Rate abgetastet und die so gebildeten Abtastwerte gespeichert werden,
- dass ein durch eine Folge von Abtastwerten gebildeter Ausschnitt des Messignals mit einem gleich langen Ausschnitt eines dem typischen Verlauf des Messignals entsprechenden Referenzsignals verglichen wird, das als Folge von Referenzwerten ebenfalls gespeichert ist,
- dass der Ausschnitt des Referenzsignals und gegebenenfalls des Messignals wiederholt um jeweils eine Stützstelle verschoben und die verschobenen Ausschnitte erneut miteinander verglichen werden,
- dass diejenige Verschiebung ermittelt wird, bei der die Kurvenformen der Ausschnitte am besten übereinstimmen,
- und dass die Laufzeit durch Korrektur des ermittelten Zeitraums entsprechend der so ermittelten Verschiebung der Ausschnitte ermittelt wird.

Eine erfindungsgemässe Vorrichtung zur Lösung der gestellten Aufgabe ist in Anspruch 7 definiert.

Die Erfindung benutzt also ebenso wie die bekannten Anordnungen die relativ ungenaue Messung des Zeitraums, der zwischen dem Aussenden des Ultraschallimpulses und dem Erreichen eines Schwellenwertes durch das Messignal verstreicht. Dieser relativ ungenaue Wert wird jedoch korrigiert.

Zur Korrektur wird ein Referenzsignal herangezogen, das den typischen zeitlichen Verlauf des Messignals im ungestörten Betrieb und bei mittlerer akustischer Dämpfung darstellt. Das Referenzsignal kann beispielsweise dadurch gewonnen werden, dass zwischen der Sender- und der Empfängeranordnung ein akustisch homogenes Medium plaziert wird, durch das der Ultraschallimpuls eine mittlere Dämpfung erfährt. Das dabei gewonnene Messignal wird abgetastet und in einem Speicher gespeichert. Die Messung wird unter gleichbleibenden Bedingungen mehrfach wiederholt und es wird der Mittelwert derjenigen Abtastwerte gebildet, die zum selben Zeitpunkt (bezogen auf das Aussenden des Ultraschallimpulses) aufgetreten sind, damit der Einfluss von Störungen (Rauschen usw.) eliminiert wird. Diese Mittelwerte bilden die Referenzwerte, und die Folge der Referenzwerte stellt das Referenzsignal dar. Das so gebildete Referenzsignal kann ein für allemal beibehalten werden. Unter Umständen kann es jedoch auch zweckmässig sein, mehrere Referenzsignale für hinsichtlich Geschwindigkeit und Dämpfung stark voneinander abweichende Medien zu speichern und bei der Laufzeitbestimmung das geeignetste zu benutzen.

Die Korrektur basiert auf der Überlegung, dass die Kurvenform des gespeicherten Ausschnittes des Messignals bei passender zeitlicher Verschiebung ungefähr der Kurvenform des Referenzsignals entsprechen muss. Diese zeitliche Verschiebung wird bestimmt, indem die Ausschnitte Schritt für Schritt jeweils um eine Stützstelle gegeneinander verschoben werden und diejenige Verschiebung ermittelt wird, bei der sich die beste Übereinstimmung zwischen den Ausschnitten des Messignals und des Referenzsignals ergibt. Wenn dabei der Ausschnitt des Messignals zu späteren Zeitpunkten oder der Ausschnitt des Referenzsignals zu früheren Zeitpunkten verschoben wird, muss die Verschiebung von dem eingangs ermittelten Zeitraum subtrahiert werden; andernfalls muss sie addiert werden. Gegebenenfalls kann es noch erforderlich sein, zu dem so korrigierten Wert einen vom Geräteaufbau und dem gewählten Verfahren bestimmten Wert zu addieren.

Wenn das Referenzsignal mit derselben Stützstellendichte gespeichert ist wie das Messignal, entspricht die Genauigkeit, mit der die Laufzeit bestimmt werden kann, dem Kehrwert der Abtastrate. Um also die geforderte Genauigkeit zu erreichen, müsste das Messignal alle 20 ns – oder häufiger – abgetastet werden. Dazu wären sehr schnelle Digital-Analog-Wandler erforderlich. Ausserdem wäre die Zahl der Stützstellen, die gespeichert werden müssten, um einen genügend langen Ausschnitt zu speichern, relativ gross, was die Zeitdauer für die Bestimmung der geeignetsten Verschiebung heraufsetzen würde.

Nach einer bevorzugten Ausgestaltung der Erfindung ist daher vorgesehen, dass das Referenzsignal mit einer um den Faktor K grösseren Stützstellendichte gespeichert ist als das Messignal und dass die Teilfolge der jeweils den Ausschnitt bildenden Referenzwerte aus den Referenzwerten gebildet wird, zwischen denen in der Folge K − 1 andere Referenzwerte liegen. Die Genauigkeit ist dabei grösser, weil die damit möglichen Verschiebungen zwischen dem Messignal und dem Referenzsignal kleiner sein können; die Genauigkeit wird dabei bestimmt durch den Kehrwert des Produktes aus der Abtastrate und dem Faktor K. Die Zahl der Stützstellen, mit denen das Messignal gespeichert wird, kann dabei ebenfalls um den Faktor K verringert werden.

Für das erfindungsgemässe Verfahren ist es nur erforderlich, dass die Abtastwerte gespeichert werden, die nach der Ankunft des Ultraschallimpulses beim Ultraschallwandler, der das Messignal erzeugt, auftreten. Der vorherige Teil des Ausgangssignals des Ultraschallwandlers, der ausschliesslich durch Störungen und Rauschen gebildet wird, muss nicht gespeichert werden; er braucht auch nicht abgetastet zu werden.

Ein Ausführungsbeispiel der Erfindung wird nachstehend anhand der Zeichnung näher erläutert. Es zeigen

Fig. 1a bis 1d den zeitlichen Verlauf eines typischen Messignals und des Referenzsignals für die beiden angegebenen Lösungen,

Fig. 2 eine erfindungsgemässe Anordnung zur Ultraschalluntersuchung,

Fig. 3 ein Flussdiagramm zur Bestimmung von 1*

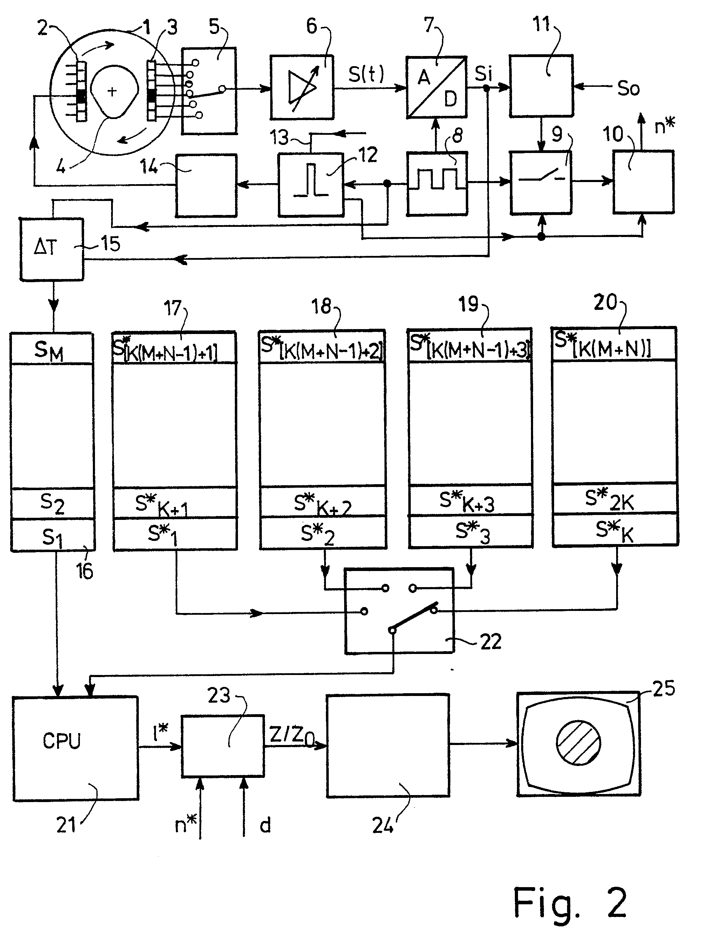

In Fig. 2 ist ein zylindrischer Behälter 1 (in der Draufsicht) dargestellt, der mit einer geeigneten Flüssigkeit, z.B. Wasser, gefüllt ist. In diesem Behälter befindet sich eine Vielzahl (z.B. 60) von auf einer Geraden nebeneinander angeordneten Ultraschallsendern 2. Weiter befindet sich in dem Behälter 1 eine entsprechende Anzahl von Ultraschallempfängern 3, die ebenfalls auf einer Geraden nebeneinander angeordnet sind, wobei die Reihen 2 und 3 parallel zueinander verlaufen. Zwischen beiden Reihen befindet sich der Untersuchungsbereich, und in diesem Bereich wird das zu untersuchende Objekt 4 – mit einer derartigen Untersuchungsanordnung werden vorzugsweise Mammauntersuchungen durchgeführt – angeordnet.

Zur Bestimmung der Laufzeit wird ein Ultraschallsender kurzzeitig erregt. Der Ultraschallimpuls durchquert den Untersuchungsbereich und damit das Objekt 4 und wird von dem gegenüberliegenden Ultraschallempfänger in ein elektrisches Signal umgesetzt. Dieser Vorgang wiederholt sich, wobei jedoch benachbarte Ultraschallwandler senden bzw. empfangen, bis alle Wandler einmal empfangen bzw. gesendet haben. Anschliessend werden die Ultraschallsende- und die -empfängeranordnung 2 bzw. 3, die auf nicht näher dargestellte Weise mechanisch miteinander gekoppelt sind, um einen kleinen Winkelbetrag um eine zur Zeichenebene senkrechte Achse gedreht und der beschriebene Vorgang wiederholt sich usw. Nachdem auf diese Weise ein Winkelbereich von mindestens 180° erfasst ist, werden die Ultraschallsender 2 gemeinsam mit den Ultraschallempfängern 3 in Richtung senkrecht zur Zeichenebene verschoben, so dass auch tiefer (oder höher) gelegene Schichten erfasst werden können.

Die Ausgänge der Ultraschallempfänger 3 sind über einen Multiplexer 5 mit dem Eingang eines Verstärkers 6 mit elektronisch einstellbarer Verstärkung verbunden, dessen Ausgangssignal einem Analog-Digital-Wandler 7 zugeführt wird. Die Verstärkung des Verstärkers 6 wird so gewählt, dass sein Ausgangssignal s wenigstens annähernd in den Amplitudenbereich fällt, den der Analog-Digital-Wandler bearbeiten kann. Zu diesem Zweck kann die Verstärkung beispielsweise in Abhängigkeit von dem Spitzenwert der vorangegangenen Messung korrigiert werden, weil bei dieser vorangegangenen Messung die Dämpfung in der Regel nicht wesentlich anders ist.

Der Takteingang des Analog-Digital-Wandlers 7 ist mit dem Ausgang eines Taktgenerators 8 verbunden, der ein impulsförmiges Taktsignal mit einer Periodendauer von z.B. 60 ns erzeugt. Der Ausgang des Taktgenerators 8 ist ausserdem über einen steuerbaren Schalter 9 mit dem Eingang eines Zählers 10 verbunden. Der steuerbare Schalter 9 wird von einem Komparator 11 gesteuert, der die vom Analog-Digital-Wandler 7 gelieferten Binärzahlen mit einer Binärzahl $s_0$ vergleicht, die so gewählt ist, dass sie einem Abtastwert entspricht, dessen Grösse dicht oberhalb des Störpegels liegt. Sobald einer der Abtastwerte $s_i$ grösser ist als der Wert $s_0$, wird der Schalter 9 geöffnet, und er bleibt bis ans Ende der Laufzeitmessung geöffnet.

Der Taktgenerator 8 ist ausserdem mit dem Triggereingang eines Startimpulserzeugers 12 verbunden, der einen (einzigen) Startimpuls erzeugt, wenn an seinem Starteingang 13 ein entsprechendes Signal anliegt und gleichzeitig an seinem Triggereingang die Vorderflanke eines Taktimpulses auftritt. Der Startimpuls aktiviert eine Erregerschaltung 14, die einen Impuls zur Erregung des für die Laufzeitmessung jeweils vorgesehenen Ultraschallsenders erzeugt. Ausserdem wird durch diesen Startimpuls der Schalter 9 geschlossen und der Zähler 10 zurückgestellt. Der Zählerstand ist mithin in jedem Augenblick dem seit dem Startimpuls bzw. dem Aussenden des Ultraschallimpulses verstrichenen Zeitraum proportional, und der Zählerstand $n^*$ nach dem Öffnen des Schalters durch den Komparator 11 ist proportional dem Zeitraum zwischen dem Aussenden des Ultraschallimpulses und dem Zeitpunkt, in dem einer der Abtastwerte den Wert $s_0$ erreicht oder überschritten hat.

Die Abtastwerte $s_i$ des Analog-Digital-Wandlers werden ausserdem dem Eingang einer digitalen Verzögerungsleitung 15 zugeführt, die als Schieberegister ausgebildet ist und deren Ausgangssignal gegenüber dem Eingangssignal um soviel Takte T verzögert ist, wie das Schieberegister Speicherplätze zur Aufnahme von Abtastwerten aufweist. Wie noch später erläutert wird, kann unter bestimmten Voraussetzungen die digitale Verzögerungsleitung 15 entfallen. Der Ausgang der digitalen Verzögerungsleitung ist mit dem Dateneingang eines Schieberegisters 16 mit M Speicherplätzen (z.B. $M = 7$) verbunden, dessen nicht näher dargestellter Takteingang ebenfalls von den Taktimpulsen des Taktgenerators 8 gesteuert wird. Nach dem Ansprechen des Komparators 11 bzw. nach dem Öffnen des Schalters 9 werden noch M Abtastwerte der digitalen Verzögerungsleitung zugeführt, und M Abtastwerte werden in das Schieberegister 16 eingeschrieben. Wenn die digitale Verzögerungsleitung 15 nicht vorhanden wäre, wären somit die Abtastwerte $s_1 \ldots s_M$ die ersten M Abtastwerte nach dem Erreichen bzw. Überschreiten des Schwellenwertes $s_0$. Hat das die digitale Verzögerungsleitung 15 bildende Schieberegister jedoch Speicherplätze für p-Abtastwerte $s_i$ ($p < M$), dann sind die Abtastwerte $s_1 \ldots s_{p-1}$ die letzten p–1 Abtastwerte vor dem Erreichen des Schwellenwertes, während $s_p$ der erste Abtastwert ist, der grösser ist als $s_0$, und die übrigen Werte die zeitlich darauffolgenden Abtastwerte sind. Nachdem die erwähnten M Abtastwerte in das Schieberegister 16 eingelesen worden sind, wird dieser Vorgang beendet. Dazu kann beispielsweise ein nicht näher dargestellter Zähler vorhanden sein, der durch das Ausgangssignal des Komparators 11 rückgestellt wird und der z.B. die Taktleitung zwischen dem Taktgenerator 8 und der digitalen Verzögerungsschaltung 15 unterbricht.

Danach wird das Schieberegister 16 im Ring gekoppelt, so dass bei einer Zuführung eines Taktimpulses jeder Abtastwert in den Speicherplatz gebracht wird, den der bei der Abtastung unmittelbar vorhergehende Abtastwert innehatte, d.h. der Abtastwert $s_M$ gelangt auf den Speicherplatz, den vorher der Abtastwert $s_{M-1}$ belegt hatte, $s_2$ gelangt auf den Speicherplatz von $s_1$, und $s_1$ gelangt auf den Speicherplatz von $s_M$. Das Schieberegister 16 kann auch durch einen Schreib-Lese-Speicher gebildet werden, dessen Adressenzähler in bekannter Weise so gesteuert wird, dass sich nach aussen hin die Funktion eines Schieberegisters ergibt. Dabei vertauschen die Abtastwerte allerdings nicht ihre Speicherplätze.

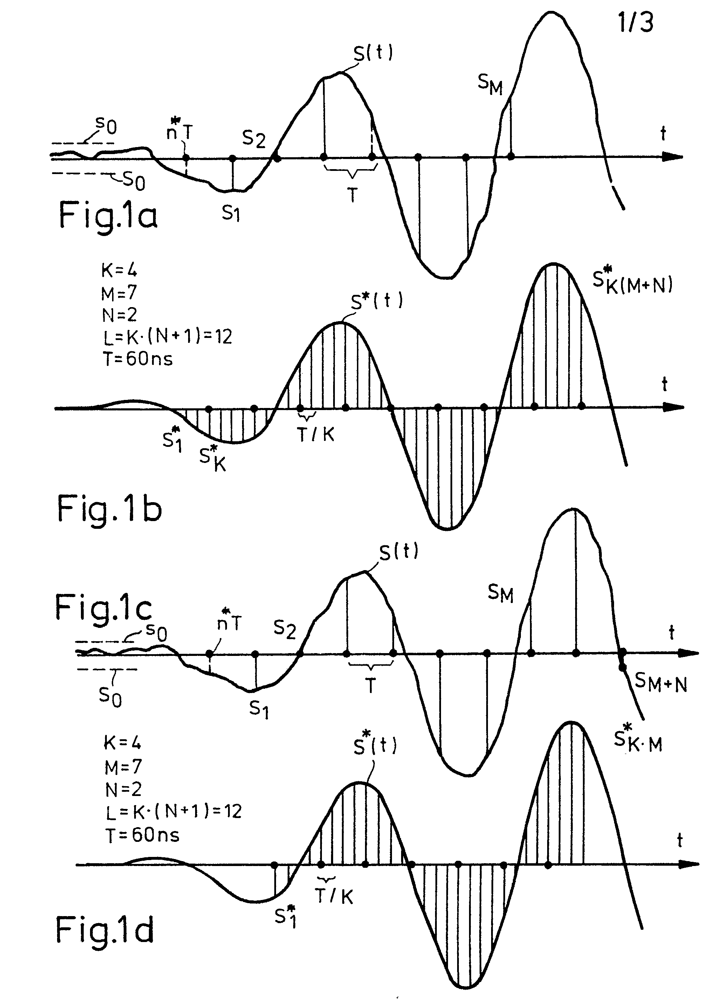

Zur Erläuterung der weiteren Signalverarbeitung wird zunächst auf Fig. 1a und Fig. 1b verwiesen. Fig. 1a zeigt den zeitlichen Verlauf des Signals s am Ausgang des Verstärkers 6. Die gestrichelten Linien $s_0$ stellen den Störpegel dar, der dem Signal überlagert ist. Dieses Signal wird mit der Abtastrate $1/T$ ($T = 60$ ns) abgetastet, d.h. alle 60 ns wird ein Abtastwert erzeugt, und zwar auch schon, bevor der erste negative Teil des Signals s den negativen Schwellwert $s_0$ überschreitet; mit «Überschreiten» ist dabei gemeint, dass der Betrag von s grösser ist als der Betrag von $s_0$. Von der Aussendung des Ultraschallimpulses bis zu diesem Zeitpunkt ist dann der Zeitraum $n^*T$ verstrichen, und $n^*$ stellt somit den Zählerstand des Zählers 10 dar. Es werden danach noch M weitere Abtastwerte $s_1 \ldots s_M$ erfasst und in der geschilderten Weise in das Schieberegister 16 eingelesen. In dem beschriebenen Fall ist die Verzögerungsleitung 15 nicht erforderlich.

Die Abtastrate T muss dabei auf die Ultraschallschwingung $s(t)$ so abgestimmt sein, dass das Signal s zwischen seinen Nulldurchgängen mehrmals abgetastet werden kann. Bei dem angegebenen Wert von T (60 ns) kann die Frequenz der Ultraschallschwingung beispielsweise 3,5 MHz betragen. M, d.h. die Zahl der Abtastwerte, ist dabei so gewählt, dass ein repräsentativer Teil des Signals s erfasst werden kann. Wie schon erwähnt, ist $M = 7$ ein geeigneter Wert.

In Fig. 1b ist der zeitliche Verlauf des Referenzsignals $s^*$ dargestellt. Auch von diesem Signal ist ein repräsentativer Teil abgetastet und die abgetasteten Werte – die hier als Referenzwerte bezeichnet sind – sind in einer Speicheranordnung abgelegt. Jedoch ist die Stützstellendichte, d.h. die Zahl der Referenzwerte pro Zeiteinheit, hier um den Faktor K grösser, wobei K eine ganze Zahl ist, z.B. 4. Dies entspricht einer Abtastrate von $K/T$, d.h. das Referenzsignal wird im zeitlichen Abstand von 15 ns abgetastet.

Ein weiterer Unterschied zu dem Messignal besteht darin, dass der Abschnitt des Referenzsignals, für den Referenzwerte gespeichert sind, grösser ist als der entsprechende Zeitraum beim Messignal s. Die Zahl der Referenzwerte beträgt $K(M+N)$, wobei N eine ganze Zahl ist, die den Maximalwert der Korrektur bestimmt und z.B. 2 beträgt.

Der Wert $n^*T$, der dem Zeitraum zwischen dem Aussenden des Ultraschallimpulses und dem Überschreiten des Schwellwertes $s_0$ darstellt, entspricht der tatsächlichen Laufzeit des Ultraschallimpulses nur näherungsweise – zum einen aus den eingangs dargelegten Gründen und zum anderen aufgrund der relativ grossen Dauer einer Abtastperiode ($T = 60$ ns). Um diesen relativ ungenauen Wert zu korrigieren, wird nun das Referenzsignal relativ zum Messignal (oder umgekehrt) «verschoben», bis die einander entsprechenden Signalabschnitte zusammenfallen, und der Wert $n^*T$ wird entsprechend dieser «Verschiebung» korrigiert.

Die Ermittlung der Grösse dieser Korrektur läuft darauf hinaus, eine Folge von Referenzwerten zu finden, die möglichst genau den Abtastwerten $s_1 \ldots s_M$ proportional ist. Die Genauigkeit dieser Korrektur ist durch den Wert T/K bestimmt, d.h. die Laufzeit kann bis auf den Wert T/K = 15 ns genau bestimmt werden, obwohl das Messignal nur alle 60 ns abgetastet wird.

Zur Bestimmung der Grösse der Korrektur wird zunächst eine Funktion F(a,l) gebildet nach der Beziehung

$$F(a,l) = \sum_{m=1}^{M} (as_m - s^{\cdot}_{K(m+N)+1-l})^2 \qquad (1)$$

Der Wert von F(a,l) stellt ein Mass für die Abweichung des durch die Abtastwerte $s_1 \ldots s_M$ definierten und mit dem Faktor a skalierten Abschnittes des Messignals von einem entsprechend langen Abschnitt im Referenzsignal dar, der durch den Parameter l definiert ist. Der Faktor a trägt dabei der Tatsache Rechnung, dass bei festem l die «Passgenauigkeit» auch noch von der Amplitude des Messignals abhängig ist. Man muss sich jedoch auf positive Werte beschränken.

Gesucht ist nun der Wert l\*, für welchen die Funktion F(a,l) ihr Minimum annimmt. Dazu kommt man durch folgende Überlegung: Es ist

$$F(a,l) = a^2 \cdot A - 2a \cdot B(l) + C(l) \qquad (2),$$

wobei

$$A = \sum_{m=1}^{M} s_m^2 \qquad (3)$$

$$B(l) = \sum_{m=1}^{M} s_m \cdot s^{\cdot}_{K(m+N)+1-l} \qquad (4)$$

$$C(l) = \sum_{m=1}^{M} (s^{\cdot}_{K(m+N)+1-l})^2 \qquad (5).$$

Bei festem l wird F(a,l) als Funktion von a höchstens dort minimal, wo

$$\frac{\delta F(a,l)}{\delta a} = 0.$$

Dies ist bei

$$a(l) = B(l)/A \qquad (6)$$

der Fall.

Setzt man den Wert a(l) anstelle von a in Gleichung (2) ein und bezeichnet den so erhaltenen Wert mit G(l), ergibt sich

$$G(l) = -B^2(l)/A + C(l) \qquad (7).$$

Es genügt also, den Wert von l zu finden, für den G(l) ein absolutes Minimum hat. Man beachte aber, dass G(l) Nebenminima haben kann. Der vorgesehene Verschiebebereich ist aber so gewählt, dass in ihm neben dem gesuchten Hauptminimum nur noch höchstens ein Nebenminimum liegt. Das Hauptminimum ist dann durch die Zusatzbedingung: a > 0 oder nach (6) B(l) > 0 charakterisiert. Zur Berechnung von G(l) muss nach Gleichung (3) aus den Abtastwerten die Grösse A berechnet werden sowie für jedes l aus diesen Werten und den korrespondierenden Referenzwerten die Grösse B(l). Die Grösse C(l) ist unabhängig vom Messignal, und sie kann daher bereits in einem geeigneten Speicher gespeichert sein. Aus den so gebildeten Werten von A, B(l) und C(l) ist dann G(l) nach Gleichung (7) zu berechnen und der Wert l\*, für den G(l) ein Minimum aufweist, wird zur Korrektur benutzt, indem zu dem Ausdruck n\*T der Wert l\* T/K addiert wird. Der Wert G(1\*), d.h. das Minimum der Funktion G(l), ist ein Mass für die Güte der Anpassung zwischen den Abtastwerten und der durch l\* definierten Folge von Referenzwerten: je kleiner der stets nichtnegative Wert G(1\*) ist, desto besser entsprechen sich das Messignal und das Referenzsignal und desto sicherer ist der gefundene Wert l\*.

Die geschilderte Berechnung kann jedoch noch weiter vereinfacht werden, wenn man sich vor Augen hält, dass der Wert A unabhängig von l ist und C(l) sich nur wenig ändert, wenn l um 1 erhöht oder erniedrigt wird. Dann hat nämlich die Funktion G(l) an der gleichen Stelle ein Minimum mit der Zusatzbedingung B(l) > 0, an der B(l) ein Maximum aufweist und es ist nur erforderlich, dass Maximum von B(l) zu bestimmen, was mit geringerem rechnerischem Aufwand möglich ist. Die Grösse B(l) stellt überdies eine digitale Version der sogenannten Kurzzeit-Kreuzkorrelationsfunktion (vgl. Woschni «Informationstechnik» 2. Auflage, 1976, VEB-Verlag Technik Berlin, Seite 161) zwischen dem Messignal und dem Referenzsignal dar, die ein Mass zwischen der Korrelation der beiden Signale s und s\* ist. Allerdings sagt die Grösse des Maximalwertes B(l\*) nichts Eindeutiges über die Güte der Anpassung zwischen den Abtastwerten und den Referenzwerten aus, weil B(l\*) ausser von dieser Anpassung auch von den Schwankungen der Amplitude des Analogsignals am Eingang des Analog-Digital-Wandlers 7 abhängt. Nachdem dasjenige l\* gefunden ist, für welches B(l) maximal wird, kann man noch G(l\*) ausrechnen und als Gütemass für die Passgenauigkeit heranziehen. Dies ist etwas weniger rechenaufwendig, als von vornherein alle Werte G(l) zu berechnen.

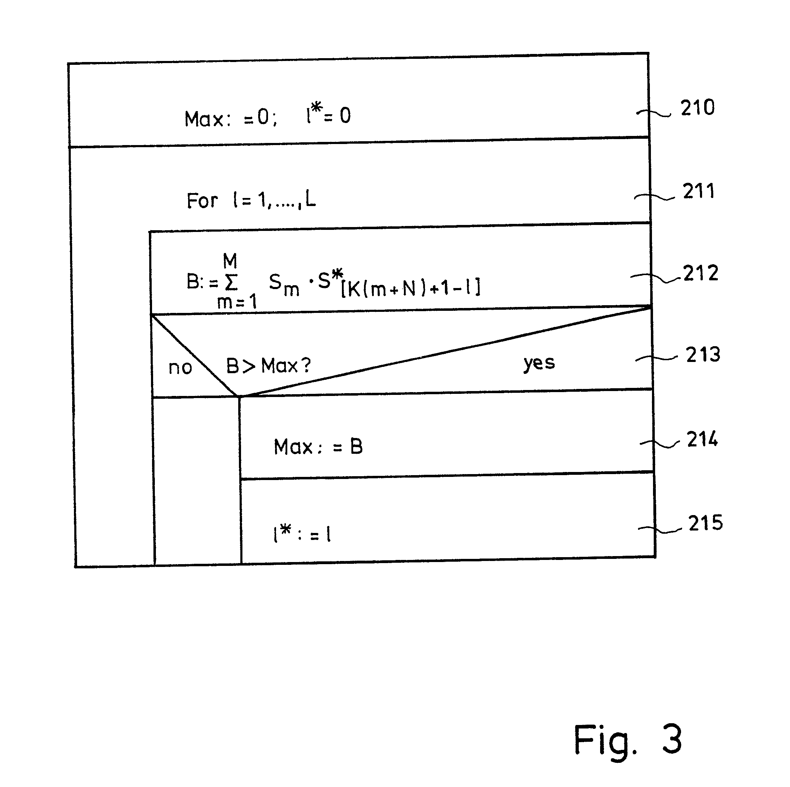

Im folgenden wird die Ermittlung des Maximalwertes von B(l) anhand von Fig. 2 sowie anhand des Flussdiagramms in Fig. 3 näher erläutert.

Die Referenzwerte sind in vier Speicheranordnungen 17, 18, 19 und 20 gespeichert, die als Schieberegister ausgebildet sein können, vorzugsweise aber durch einen Nur-Lesespeicher (PROM) gebildet werden, dessen Adressenzähler so gesteuert wird, dass die Werte nacheinander aufgerufen werden können. Jeder der Speicher 17...20 weist mindestens M + N-Speicherplätze auf, im angegebenen Beispiel also mindestens 9 Speicherplätze. Im Speicher 17 ist der erste Referenzwert und jeder K-te (vierte) darauffolgende Referenzwert gespeichert, d.h. die Referenzwerte $s^*_1$, $s^*_{K+1}$ . . . , $s^*_{K(M + N = 1) + 1}$. Im zweiten Speicher 18 sind die Werte $s^*_2$ . . . $s^*_{K(M + N - 1) + 2}$ gespeichert und im K-ten Speicher, d.h. im vierten Speicher 20, die Werte $s^*_K$ . . . $s^*_{K(M + N)}$. Es ist auch möglich, sämtliche Referenzwerte in einem einzigen Speicher zu speichern; dabei sollten in Speicherplätzen mit aufeinanderfolgenden Adressen nicht aufeinanderfolgende Referenzwerte gespeichert werden, sondern nur jeder K-te Referenzwert – also z.B. $s^*_K$, $s^*_{2K}$ . . . usw. – was die Adressierung bei der späteren Verarbeitung vereinfacht. Die Ausgänge der vier Speicher sind über einen Multiplexer 22 mit einer Recheneinheit 21 verbunden. Der Recheneinheit 21 werden ausserdem die in 16 gespeicherten Werte $s_1$ . . . $s_m$ zugeführt.

Die Recheneinheit 21 bestimmt denjenigen Wert I*, für den B(I) ein Maximum aufweist. Sie kann mit Hilfe eines geeignet programmierten Microcomputers realisiert werden. Das Strukturdiagramm (Nassi-Schneiderman-Diagramm), nach dem dieser den Wert I* ermittelt, ist in Fig. 3 dargestellt. Nachdem die Werte $s_1$ . . . $s_m$ in das Schieberegister eingelesen worden sind, wird im ersten Schritt 210 der Vergleichswert Max = 0 gesetzt und ebenso der Wert I*. Der zweite Schritt 211 stellt eine Schleife dar, die L mal durchlaufen wird (L = K(N + 1) = 12). Die Schleife beinhaltet als ersten Schritt 212 die Berechnung des Wertes B – zunächst für den Wert I = 1. Dieser Schritt stellt ein Unterprogramm dar, bei dem zunächst aus dem Speicher 16 der Wert $s_1$ und aus dem Speicher 20 der Wert $s^*_{K(m + N)} = s^*_{3K}$ aufgerufen wird. Diese Werte werden miteinander multipliziert. Zu diesem Produkt wird das Produkt der Werte $s_2$ und $s^*_{4K}$ addiert usw., bis im siebten Schritt der Abtastwert $s_M$ und der Referenzwert $s^*_{K(M + N)}$ aufgerufen, miteinander multipliziert und zu der erwähnten Summe addiert werden. Sämtliche Referenzwerte werden dabei aus dem Speicher 20 aufgerufen.

Nachdem der Wert B auf diese Weise berechnet ist, wird er im Schritt 213 mit dem Vergleichswert Max verglichen. Ist der berechnete Wert B(1) grösser als der Vergleichswert Max, so wird dem Vergleichswert Max der berechnete Wert B zugewiesen (Schritt 214) und dem Wert I* wird der jeweilige Wert von I – in diesem Fall also 1 – zugewiesen (Schritt 215). Danach, bzw. wenn die Abfrage 213 ergeben hat, dass der berechnete Wert B nicht grösser ist als der Vergleichswert, kehrt die Berechnung zum ersten Schritt 212 der Programmschleife 211 zurück, wobei jedoch 1 = 2 gesetzt wird. Zur Berechnung von B werden in diesem Fall die Referenzwerte $s^*_{2K + 3}$ . . . $s^*_{K(M + N - 1) + 3}$ aus dem Speicher 19 mit den zugehörigen Werten $s_1$ . . . $s_m$ aus dem Speicher 16 multipliziert, wobei der Multiplexer den Ausgang dieses Speichers 19 mit dem Dateneingang der Recheneinheit 21 verbinden muss. Dabei wird wiederum die Abfrage 213 durchlaufen, wobei je nachdem, ob der neu berechnete Wert grösser ist als der (unter Umständen schon geänderte) Vergleichswert Max die Werte Max bzw. I* verändert werden oder nicht, wonach sich der nächste Schritt (mit I = 3) wiederholt. Die Schritte 212, 213 und gegebenenfalls 214, 215 wiederholen sich insgesamt zwölfmal.

Danach ist in der Regel das Maximum der Werte B(I) bis B(12) bestimmt und der zugehörige Wert I*. Lediglich im Falle I* = 0 (Schritt 210) wären alle B-Werte negativ gewesen. Dieses Ereignis deutet aber auf irgendeinen Fehler hin und kann daher benutzt werden, um den Einfluss dieses offensichtlich falschen Wertes auf die Rekonstruktion zu eliminieren.

Bisher wurde davon ausgegangen, dass die Grössen B(I) bzw. G(I) für alle Werte von I, d.h. für I = 1 . . . L (L = K(N + 1) ) gebildet werden müssen. Dieser Wertebereich könnte für jede Messung jedoch eingeschränkt werden, wenn die Lage eines charakteristischen Teils des Messignals s, z.B. des ersten positiven Maximums (in Fig. 1 wäre das $s_3$) bekannt ist. Dazu muss lediglich das erste positive Maximum der Folge bestimmt werden, und je nachdem, bei welchem der Werte $s_1$ . . . $s_M$ dieses Maximum liegt, wird der Bereich eingeschränkt, innerhalb dessen I variiert wird. Dadurch kann die Rechenzeit verringert werden.

Die Recheneinheit 21 kann mit Hilfe eines entsprechend programmierten Mikroprozessors realisiert werden. Dieser kann dann auch weitere Steuerfunktionen übernehmen, z.B. die Adressierung der Speicher 17 bis 20 und die Steuerung des Schieberegisters 16 und des Multiplexers 22. Auch die Erzeugung des Startimpulses auf der Leitung 13, der Vergleich der Abtastwerte $s_i$ mit dem Schwellwert $s_0$ und die Zählung der Impulse des Taktgenerators könnten mit Hilfe dieses Mikroprozessors erfolgen und gegebenenfalls kann der Mikroprozessor die Taktimpulse auch selbst erzeugen. – Die arithmetischen Operationen, die die Recheneinheit auszuführen hat, sind relativ einfach (Multiplizieren, Summieren und Vergleichen). Die Recheneinheit kann auch durch geeignete Bitslice-Prozessoren realisiert werden, welche beim gegenwärtigen Stand der Technik schneller als ein einzelner Mikroprozessor sind.

Wird zur Berechnung von B(I) oder G(I) Festkommaarithmetik benutzt, was aus Gründen der Rechengeschwindigkeit vorteilhaft ist, kann es zu Zahlenbereichsüberschreitungen bei Zwischenergebnissen kommen. Dem kann durch geeignete, sogenannte Skalierungsmassnahmen vorgebeugt werden.

In einer Summierstufe 23 wird die Laufzeit Z nach der Beziehung

$$Z = n^*T + I^*T/K - d \qquad (8)$$

gebildet. n* ist dabei der Inhalt des Zählers 10 und d eine Gerätekonstante. Der Wert dieser Konstanten, der nur einmal ermittelt werden muss, lässt sich verhältnismässig einfach bestimmen, wenn bei sonst gleichen Verhältnissen die Laufzeit bei zwei verschiedenen Abständen von Sendern und Empfängern nach Gleichung (8) bestimmt wird und der Wert d so variiert wird, dass die aufgrund von Gleichung (8) ermittelten Laufzeiten den Abständen von Ultfaschallsendern und -empfängern proportional sind. Je nach Art des benutzten Verstärkers 6 kann d zusätzlich vom jeweiligen Verstärkungsfaktor abhängen. Diese Abhängigkeit ist aber a priori bekannt und lässt sich daher leicht berücksichtigen.

Die Schallgeschwindigkeit in einem Medium hängt von dessen Temperatur ab. Bei einem flüssigen Medium, wie Wasser, ist die Abhängigkeit derart, dass die Laufzeit über eine Strecke von wenigen Zentimetern bei einer Temperaturänderung um wenige Zehntelgrad schon um mehr als den Wert T/K variieren kann, so dass die Messung allein aufgrund solcher Temperaturabhängigkeiten zu ungenau würde. Eine Möglichkeit, um derartige Fehlmessungen zu vermeiden, besteht darin, die Temperatur der Flüssigkeit in dem Behälter 1 konstant zu halten.

Man kann jedoch auch den Einfluss der Temperatur auf die Laufzeit weitgehend eliminieren. Zu diesem Zweck kann in jeder Winkelstellung der Ultraschallsender- bzw. Empfangsanordnung 2 bzw. 3 die Laufzeit $Z_0$ eines Ultraschallimpulses bestimmt werden, der durch das Untersuchungsobjekt 4 nicht beeinflusst wird, und denselben geometrischen Abstand zwischen Sender und Empfänger hat. Dies ist in der Regel bei den äusseren Ultraschallsende- und -empfangselementen der Ultraschallwandlerreihen 2 und 3 der Fall. Die Laufzeit $Z_0$, die aufgrund der dabei gemessenen Werte $n^*$ und $l^*$ nach Gleichung (8) ermittelt wird, ist praktisch in gleicher Weise von der Temperatur abhängig wie die kurze Zeit später ermittelte Laufzeit Z eines durch das Objekt 4 beeinflussten Ultraschallimpulses. Der Quotient $Z/Z_0$ ist daher ein von der Temperatur im wesentlichen unabhängiger Wert, der der Laufzeit proportional ist. Dieser Quotient wird einem Rechner 24 zugeführt, der die Brechungsindexverteilung an den einzelnen Punkten einer Schicht des untersuchten Objektes 4 berechnet. Die berechnete Verteilung wird auf einem geeigneten Wiedergabegerät 25, z.B. einem Fernsehmonitor, wiedergegeben.

Es sind einige weitere Varianten des bislang beschriebenen Verfahrens denkbar. Zum Beispiel kann man in dem Ausdruck (1) für F(a,l) den Faktor a auch an dem Referenzsignal anbringen. Dieselben Überlegungen wie oben führen dann zur Minimierung von

$$G'(l) = A - B(l)^2/C(l) \qquad (9)$$

mit der Nebenbedingung B(l) > 0. Dies ist wiederum im wesentlichen gleichwertig zur Maximierung von B(l). Die Ausdrücke A, B(l), C(l) in (9) sind dabei wie in (3), (4), (5) definiert. Anders als im Falle von G(l) gemäss (7) liefert der Wert des Minimums $G(l^*)$ keine Zuverlässigkeitsaussage.

Im bislang betrachteten Ausführungsbeispiel wurde der erste Abtastwert gesucht, der negativ ist und dem Betrage nach einen vorgegebenen Schwellwert $s_0$ überschreitet. Es kann jedoch vorteilhafter sein, stattdessen den ersten Abtastwert zu suchen, welcher positiv ist und einen positiven Schwellwert $s_0'$ überschreitet; dabei kann $s_0'$ auch grösser sein als $s_0$. Im Beispiel von Fig. 1a ist das etwa für $s_3$ der Fall. Um aber auch bei dieser Variante die Abtastwerte $s_1 \ldots s_M$ zu speichern, was wünschenswert ist, kann man die Verzögerungsleitung 15 benutzen, hier mit p = 3.

Man könnte in naheliegender Weise auch eine positive und eine negative Schwelle miteinander kombinieren und z.B. mit der Dauerspeicherung erst beginnen, wenn kurz nacheinander die negative Schwelle unter- und die positive Schwelle überschritten wurde.

Bisher wurde davon ausgegangen, dass der durch Referenzwerte erfasste Bereich des Referenzsignals grösser ist als der durch Abtastwerte erfasste Bereich des Messignals. Das erfindungsgemässe Verfahren ist jedoch auch durchführbar, wenn der durch Abtastwerte definierte Bereich des Messignals vergrössert wird, so dass z.B. M + N-Abtastwerte zur Verfügung stehen (vgl. Fig. 1c) und der entsprechende Bereich des Referenzsignals verkleinert wird, so dass nur noch K · M Referenzwerte vorhanden sind (Fig. 1d).

Auch in diesem Fall lässt sich die Übereinstimmung der ausgewählten Bereiche des Referenz- und des Messignals durch eine Funktion F(a,l) numerisch beschreiben, die der Beziehung

$$F(a,l) = \sum_{m=1}^{M} (a \cdot s_{x(m,l)} - \overset{.}{s}_{y(m,l)})^2 \qquad (10)$$

genügt.

Dabei wurde gesetzt

$$x(m,l) = m + \mathrm{int}\,((l-1)/K) \qquad (11)$$

und

$$y(m,l) = K \cdot m - (l-1) + K \cdot \mathrm{int}\,((l-1)/K) \qquad (12)$$

Dabei ist int ( ) eine Funktion, die der grössten ganzen Zahl entspricht, die nicht grösser ist als das Argument ( ) dieser Funktion.

Auch in diesem Fall nimmt die Funktion F(a,l) ihr Minimum für dasjenige l an, bei welchem der Ausdruck

$$G(l) = -B^2(l)/A(l) + C(l)$$

minimal wird, wobei jetzt

$$A(l) = \sum_{m=1}^{M} (s_{X(m,l)})^2 \qquad (13)$$

$$B(l) = \sum_{m=1}^{M} s_{x(m,l)} \cdot \overset{.}{s}_{y(m,l)} \qquad (14)$$

$$C(I) = \sum_{m=1}^{M} \overset{\cdot}{s}_{y(m,I)}{}^2 \qquad (15).$$

Auch hierbei ist die Minimierung von G(I) im wesentlichen gleichwertig zur Maximierung von B(I); auch sind analoge Varianten möglich wie bei der ersten Lösung. Die Berechnung von I* entspricht daher dem schon geschilderten Verfahren, so dass auf eine nochmalige Darstellung verzichtet werden kann.

**Patentansprüche**

1. Verfahren zur Laufzeitbestimmung eines Ultraschallimpulses, der einen Untersuchungsbereich durchsetzt und in ein elektrisches Messignal umgesetzt wird, wobei der Zeitraum (n*T) gemessen wird, der nach dem Aussenden des Ultraschallimpulses verstreicht, bis das Messignal einen vorgegebenen Wert erreicht hat, dadurch gekennzeichnet,

— dass das Messignal (s) mit einer vorgegebenen Rate (1/T) abgetastet und die so gebildeten Abtastwerte gespeichert werden,

— dass ein durch eine Folge von Abtastwerten ($s_1 \dots s_M$) gebildeter Ausschnitt des Messignals (s) mit einem gleich langen Ausschnitt (z.B. $s_{K+1} \dots s_{KM+1}$) eines dem typischen Verlauf des Messignals entsprechenden Referenzsignals (s*) verglichen wird, das als Folge von Referenzwerten ebenfalls gespeichert ist,

— dass der Ausschnitt des Referenzsignals (s*) und gegebenenfalls des Messignals (s) wiederholt um jeweils eine Stützstelle verschoben und die verschobenen Ausschnitte (z.B. $s_1 \dots s_M$; $s^*_K \dots s^*_{KM}$) erneut miteinander verglichen werden,

— dass diejenige Verschiebung (I*) ermittelt wird, bei der die Kurvenformen der Ausschnitte am besten übereinstimmen

— und dass die Laufzeit (Z) durch Korrektur des ermittelten Zeitraums (n*T) entsprechend der so ermittelten Verschiebung (I*) der Ausschnitte ermittelt wird.

2. Verfahren nach Anspruch 1, dadurch gekennzeichnet, dass das Referenzsignal (s*) mit einer um den ganzzahligen Faktor K grösseren Stützstellendichte gespeichert wird als das Messignal (s) und dass aus den Referenzwerten zur Bildung des Ausschnitts des Referenzsignals jeweils solche Werte ($s^*_K$, $s^*_{2K} \dots s^*_{K(M-1)}$, $s^*_{KM}$) ausgewählt werden, zwischen denen in der Folge K − 1 andere Referenzwerte liegen.

3. Verfahren nach einem der Ansprüche 1 oder 2, dadurch gekennzeichnet,

— dass in Abhängigkeit von I mehrere Werte G(I) nach der Beziehung

$$G(I) = -B(I)^2/A(I) + C(I)$$

gebildet werden, wobei I eine variable ganze Zahl ist und wobei

$$A(I) = \sum_{m=1}^{M} s_m{}^2$$

$$B(I) = \sum_{m=1}^{M} s_m \overset{\cdot}{s}_{y(m,I)}$$

$$C(I) = \sum_{m=1}^{M} (\overset{\cdot}{s}_{y(m,I)})^2$$

worin $s_m$ der m-te Abtastwert und $s^*_{y(m,I)}$ der y-te Referenzwert ist und wobei gilt

$$y(m,I) = K(m+N) + (1 - I,$$

worin N und K ganze Zahlen sind und die Zahl K gleich dem Quotienten aus der Stützstellendichte des Referenzsignals einerseits und des Messignals andererseits ist,

— dass der Wert I* von I ermittelt wird, für den G(I) unter der Nebenbedingung B(I) > 0 ein Minimum aufweist,

— und dass die Laufzeit Z nach der Beziehung

$$Z = n^*T + I^*T/K - d$$

gebildet wird, wobei T der Kehrwert der Abtastrate und d ein vorgegebener Apparateparameter ist und wobei n* angibt, wie oft der Kehrwert der Abtastrate in dem gemessenen Zeitraum (n*T) enthalten ist.

4. Verfahren nach einem der Ansprüche 1 oder 2, dadurch gekennzeichnet,

— dass in Abhängigkeit von I mehrere Werte G(I) nach der Beziehung

$$G(I) = -B(I)^2/A(I) + C(I)$$

gebildet werden, wobei I eine variable ganze Zahl ist und wobei

$$A(I) = \sum_{m=1}^{M} (s_{x(m,I)})^2$$

$$B(I) = \sum_{m=1}^{M} s_{x(m,I)} \cdot \overset{\cdot}{s}_{y(m,I)}$$

$$C(I) = \sum_{m=1}^{M} (\overset{\cdot}{s}_{y(m,I)})^2$$

wobei $s_x$ der x-te Abtastwert und $s^*_y$ der y-te Referenzwert ist und wobei gilt

$$x(m,I) = m + \text{int}((I-1)/K)$$

$$y(m,I) = Km + I - 1 + K\,\text{int}((I-1)/K)$$

wobei K eine ganze Zahl ist, die gleich dem Quotienten aus der Stützstellendichte des Referenzsignals einerseits und des Messignals andererseits ist,

– dass der Wert I* von I ermittelt wird, für welchen G(I) unter der Nebenbedingung B(I) > 0 ein Minimum aufweist,

– dass schliesslich die Laufzeit Z nach der Beziehung

$$Z = n^*T + I^*T/K - d$$

ermittelt wird, wobei T der Kehrwert der Abtastrate ist und d ein vorgegebener Apparateparameter ist und wobei $n^*$ angibt, wie oft der Kehrwert der Abtastrate in dem gemessenen Zeitraum ($n^*T$) enthalten ist.

5. Verfahren nach einem der vorhergehenden Ansprüche, bei dem das zu untersuchende Objekt in einen mit einer Flüssigkeit gefüllten Behälter eintaucht, dadurch gekennzeichnet, dass zur Rekonstruktion der akustischen Eigenschaften die Laufzeit Z dividiert wird durch $Z_0$, wobei $Z_0$ die Laufzeit eines Ultraschallimpulses ist, der von dem zu untersuchenden Objekt praktisch nicht beeinflusst worden ist und bei dessen Erfassung derselbe geometrische Abstand zwischen Sender und Empfänger zugrunde gelegen hat.

6. Verfahren nach einem der Ansprüche 3 oder 4, dadurch gekennzeichnet, dass die Kurzzeit-Kreuzkorrelationsfunktion B(I) des Messignals s und des Referenzsignals (s*) mit Hilfe der Abtastwerte ($s_x$) bzw. der Referenzwerte ($s^*_y$) gebildet wird und dass der Wert von I ermittelt wird, für den die Kurzzeit-Kreuzkorrelationsfunktion B(I) ein absolutes Maximum aufweist.

7. Anordnung zur Durchführung des Verfahrens nach Anspruch 3 oder 4 mit einer Senderanordnung (2) zum Erzeugen von Ultraschallimpulsen und einer Empfängeranordnung (3) zum Empfangen der Ultraschallimpulse, mit einer Detektoreinrichtung (6 . . . 11), die den Zeitraum zwischen dem Senden eines Impulses und dem Zeitpunkt bestimmt, in dem das von der Empfängeranordnung erzeugte Messignal einen vorgebbaren Wert überschreitet, mit einer Rekonstruktionseinheit (24), die aus den Laufzeiten der Ultraschallimpulse die akustischen Eigenschaften des Untersuchungsbereiches zwischen der Sende- und der Empfangsanordnung bestimmt und mit einer Wiedergabeeinheit (25) zur Darstellung des durch die Rekonstruktionseinheit ermittelten Bildes des Untersuchungsbereiches, gekennzeichnet durch

– Mittel (7) zum Abtasten des Empfangssignals (s) mit einer vorgebbaren Abtastrate (1/T),

– eine erste Speicheranordnung (16) so gebildeten Abtastwerte ($s_x$),

– eine zweite Speicheranordnung (17 . . . 20), in der die Stützstellen ($s^*_y$) eines dem typischen Verlauf des Messignals entsprechenden Referenzsignals mit einer um den Faktor K grösseren Dichte gespeichert sind,

– eine Recheneinheit zum Bestimmen eines Wertes I* von I, für den die Funktion

$$G(I) = -B(I)^2/A(I) + C(I)$$

ein Minimum hat unter der Nebenbedingung B(I) > 0 oder für den die Funktion B(I) ein Maximum hat,

– und Mittel (23) zum Bilden eines der Laufzeit entsprechenden Wertes nach der Beziehung

$$Z = n^*T + I^*T/K - d,$$

wobei für die Grössen A(I), B(I), C(I), I, K, $n^*$, T und d die in den Ansprüchen 3 bzw. 4 angegebenen Definitionen gelten.

**Revendications**

1. Procédé de mesure du temps de parcours d'une impulsion ultrasonore qui traverse une zone d'examen et qui est convertie en un signal de mesure électrique, suivant lequel on mesure l'intervalle de temps ($n^*T$), s'écoulant entre l'émission de l'impulsion ultrasonore et l'instant où le signal de mesure a atteint une valeur prédéterminée, caractérisé en ce que

– le signal de mesure (s) est échantillonné à une cadence prédéterminée (1/T) et les valeurs d'échantillonnage ainsi obtenues sont mémorisées,

– en ce qu'une partie du signal de mesure (s) formée par une série de valeurs d'échantillonnage ($s_1 . . . s_M$) est comparée avec une partie de même longueur (par exemple $s_{K+1} . . . s_{KM+1}$) d'un signal de référence (s*) correspondant à l'allure typique du signal de mesure et également mémorisé sous la forme d'une série de valeurs de référence,

– en ce que la partie du signal de référence (s*) et, le cas échéant, du signal de mesure (s) est décalée à plusieurs reprises d'un point d'échantillonnage et les parties de signal décalées (par exemple $s_1 . . . s_M$; $s^*_K . . . s^*_{KM}$) sont à nouveau comparées,

– en ce qu'est déterminé celui (I*) des décalages dans lequel les formes de courbe des parties de signal correspondent le mieux

– et en ce que le temps de parcours (Z) est déterminé par correction de l'intervalle de temps mesuré ($n^*T$) en fonction du décalage (I*) ainsi déterminé des parties de signal.

2. Procédé selon la revendication 1, caractérisé en ce que le signal de référence (s*) est mémorisé à une densité de points d'échantillonnage qui est d'un facteur entier K plus grande que celle du signal de mesure (s) et en ce que, parmi les valeurs de référence, pour la formation de la partie du signal de référence, sont choisies chaque fois des valeurs ($s^*_K$, $s^*_{2K} . . . s^*_{K(M-1)}$, $s^*_{KM}$) entre lesquelles sont situées d'autres valeurs de référence dans la série K − 1.

3. Procédé selon la revendication 1 ou 2, caractérisé en ce que,

– en fonction de 1, plusieurs valeurs G(I) sont formées selon la relation

$$G(I) = -B(I)^2/A(I) + C(I)$$

où I est un entier variable et où

$$A(I) = \sum_{m=1}^{M} s_m^2$$

$$B(l) = \sum_{m=1}^{M} s_m \, \dot{s}^*_{y(m,l)}$$

$$C(l) = \sum_{m=1}^{M} (\dot{s}^*_{y(m,l)})^2$$

où $s_m$ est la $m^{\text{ième}}$ valeur d'échantillonnage et $s^*_{y(m,l)}$ la $y^{\text{ième}}$ valeur de référence, étant entendu que

$$y(m,l) = K(m+N) + 1 - l$$

où N et K sont des nombres entiers et le nombre K est égal au quotient de la densité de points d'échantillonnage du signal de référence d'une part et du signal de mesure d'autre part,

– en ce que la valeur $l^*$ est déduite de $l$, valeur pour laquelle $G(l)$ présente un minimum sous la condition secondaire $B(l) > 0$

– et en ce que le temps de parcours Z est obtenu selon la relation

$$Z = n^* T + l^* T/K - d$$

où T est la valeur réciproque de la cadence d'échantillonnage et d est un paramètre d'appareil prédéterminé et $n^*$ indique le nombre de fois que la valeur réciproque de la cadence d'échantillonnage est contenue dans l'intervalle de temps mesuré $(n^* T)$.

4. Procédé selon la revendication 1 ou 2, caractérisé en ce que,

– en fonction de $l$, plusieurs valeurs $G(l)$ sont formées selon la relation

$$G(l) = -B(l)^2/A(l) + C(l)$$

où $l$ est un entier variable et où

$$A(l) = \sum_{m=1}^{M} (s_{x(m,l)})^2$$

$$B(l) = \sum_{m=1}^{M} s_{x(m,l)} \cdot \dot{s}^*_{y(m,l)}$$

$$C(l) = \sum_{m=1}^{M} (\dot{s}^*_{y(m,l)})^2$$

où $s_x$ est la $x^{\text{ième}}$ valeur d'échantillonnage et $s^*_y$ est la $y^{\text{ième}}$ valeur de référence et où

$$x(m,l) = m + \text{int}\,((l-1)/K)$$

$$y(m,l) = Km + 1 - l + K\,\text{int}\,((l-1)/K)$$

K étant un entier égal au quotient de la densité de points d'échantillonnage du signal de référence d'une part et du signal de mesure d'autre part,

– en ce que la valeur $l^*$ est déduite de $l$, alors que pour cette valeur $l^*$, la valeur $G(l)$ présente un minimum sous la condition secondaire $B(l) > 0$ et

– en ce que, finalement, le temps de parcours est déterminé selon la relation

$$Z = n^* T + l^* T/K - d$$

où T est la valeur réciproque de la cadence d'échantillonnage et d est un paramètre d'appareil prédéterminé et où $n^*$ indique le nombre de fois que la valeur réciproque de la cadence d'échantillonnage est contenue dans l'intervalle de temps mesuré $(n^* T)$.

5. Procédé selon l'une quelconque des revendications précédentes, suivant lequel l'objet à examiner est plongé dans un bac rempli d'un liquide, caractérisé en ce que pour la reconstruction des propriétés acoustiques, le temps de parcours Z est divisé par $Z_0$, cette valeur $Z_0$ étant le temps de parcours d'une impulsion ultrasonore qui n'est pratiquement pas influencée par l'objet à examiner et lors de la détermination de laquelle on s'est basé sur la même distance géométrique entre l'émetteur et le récepteur.

6. Procédé selon la revendication 3 ou 4, caractérisé en ce que la fonction de corrélation mutuelle instantanée $B(l)$ du signal de mesure s et du signal de référence $(s^*)$ est formée à l'aide des valeurs d'échantillonnage $(s_x)$ et des valeurs de référence $(s^*_y)$ et en ce qu'est déterminée la valeur de $l$ pour laquelle la fonction de corrélation mutuelle instantanée $B(l)$ présente un maximum absolu.

7. Dispositif de mise en œuvre du procédé selon la revendication 3 ou 4, muni d'un émetteur (2) pour engendrer des impulsions ultrasonores, d'un récepteur (3) pour recevoir les impulsions ultrasonores, d'un dispositif détecteur (6 … 11) qui détermine l'intervalle de temps compris entre l'instant d'émission d'une impulsion et l'instant où le signal de mesure engendré par le récepteur dépasse une valeur prédéterminée, d'une unité de reconstruction (24) qui déduit des temps de parcours des impulsions ultrasonores les propriétés acoustiques de la zone d'examen entre l'émetteur et le récepteur, et d'une unité d'affichage (25) pour afficher l'image de la zone d'examen, déterminée par l'unité de reconstruction, caractérisé par

– des moyens (7) d'échantillonnage du signal de réception (s) à une cadence d'échantillonnage prédéterminée (1/T),

– un premier dispositif (16) de mémorisation des valeurs d'échantillonnage ainsi formées $(s_x)$,

– un second dispositif (17 … 20) de mémorisation dans lequel les points d'échantillonnage $(s^*_y)$ d'un signal de référence correspondant à l'allure typique du signal de mesure sont mémorisés à une densité supérieure d'un facteur K,

– une unité de calcul pour déterminer une valeur $l^*$ de $l$ pour laquelle la fonction

$$G(l) - B(l)^2/A(l) + C(l)$$

a un minimum sous la condition secondaire $B(I) > 0$ ou pour laquelle la fonction $B(I)$ a un maximum,

– des moyens (23) de formation d'une valeur correspondant au temps de parcours selon la relation

$$Z = n^*T + I^*T/K - d,$$

les grandeurs $A(I)$, $B(I)$, $C(I)$, $I$, $K$, $n^*$, $T$ et $d$ répondant aux définitions indiquées dans les revendications 3 et 4.

## Claims

1. A method of determining the time-of flight of an ultrasonic pulse which passes through an examination zone and which is converted into an electric measurement signal, the period of time $(n^*T)$ being measured which expires between the instant of transmission of the ultrasonic pulse and the instant at which the measurement signal reaches a predetermined value, characterized in that

– the measurement signal (s) is sampled at a predetermined rate $(1/T)$, the sampling values thus formed being stored,

– a section of the measurement signal (s) which is formed by a series of sampling values $(s_1 \ldots s_M)$ is compared with an equally long section (for example, $s_{K+1} \ldots s_{KM+1}$) of a reference signal $(s^*)$ which corresponds to the typical variation of the measurement signal and which is also stored in the form of a series of reference values,

– the section of the reference signal $(s^*)$ and possibly that of the measurement signal (s) is repeatedly shifted over each time one sampling point, the shifted sections (for example, $s_1 \ldots s_M$; $s^*_K \ldots s^*_{KM}$) being compared again,

– the shift $(I^*)$ is determined where the correspondence of the curves of the sections is best, and

– the time-of-flight (Z) is determined by correction of the measured period of time $(n^*T)$ in accordance with the shift $(I^*)$ of the sections thus determined.

2. A method as claimed in Claim 1, characterized in that the reference signal $(s^*)$ is stored with a sampling point density which is an integer factor K larger than that of the measurement signal (s), for the formation of the section of the reference signal there being chosen, each time from the reference values $(s^*_K, s^*_{2K} \ldots s^*_{K(M-1)}, s^*_{KM})$, those values wherebetween $K-1$ other reference values are situated in the series.

3. A method as claimed in Claim 1 or 2, characterized in that

– in dependence of $I$ several values $G(I)$ are formed in accordance with the relation

$$G(I) = - B(I)^2/A(I) + C(I)$$

in which $I$ is a variable integer number and in which

$$A(I) = \sum_{m=1}^{M} s_m^2$$

$$B(I) = \sum_{m=1}^{M} s_m \, s^*_{y(m,I)}$$

$$C(I) = \sum_{m=1}^{M} (s^*_{y(m,I)})^2$$

in which $s_m$ is the $m^{th}$ sampling value and $s^*_{y(m,I)}$ is the $y^{th}$ reference value, and

$$(y(m,I) = K(m+N) + 1 - I,$$

in which N and K are integer numbers and the number K is equal to the quotient of the sampling point density of the reference signal on the one hand and that of the measurement signal on other hand,

– the value $I^*$ being determined from $I$ for which $G(I)$ has a minimum subject to the subsidiary condition $B(I) > 0$,

– the time-of-flight Z being formed in accordance with the relation

$$Z = n^*T + I^*T/K - d,$$

in which T is the reciprocal value of the sampling rate, d is a predetermined apparatus parameter, and $n^*$ indicates how often the reciprocal value of the sampling rate is comprised in the measured period of time $(n^*T)$.

4. A method as claimed in any of the Claims 1 or 2, characterized in that

– in dependent of $I$, several values $G(I)$ are formed in accordance with the relation

$$G(I) = - B(I)^2/A(I) + C(I)$$

in which $I$ is a variable integer number and in which

$$A(I) = \sum_{m=1}^{M} (s_{x(m,I)})^2$$

$$B(I) = \sum_{m=1}^{M} s_{x(m,I)} \cdot s^*_{y(m,I)}$$

$$C(I) = \sum_{m=1}^{M} (s^*_{y(m,I)})^2$$

in which $s_x$ is the $x^{th}$ sampling value and $s^*_y$ is the $y^{th}$ reference value, and for which

$$x(m,I) = m + \text{int} \, ((1-I)/K)$$

$$y(m,l) = Km + 1 - l + K \text{ int} ((1 - l)/K)$$

K being an integer number equal to the quotient of the sampling point density of the reference signal on the one hand and that of the measurement signal on the other hand,

– the value $l^*$ being determined from $l$ for which $G(l)$ has a minimum subject to the subsidiary condition $B(l) > 0$,

– finally the time-of-flight Z being determined in accordance with the relation

$$Z = n^*T + 1^*T/K - d$$

in which T is the reciprocal value of the sampling rate, d is a predetermined apparatus parameter, and $n^*$ indicates how often the reciprocal value of the sampling rate is comprised in the measured period of time $(n^*T)$.

5. A method as claimed in any one of the preceding Claims, in which the object to be examined is immersed in a reservoir filled with a liquid, characterized in that for the reconstruction of the acoustic properties the time-of-flight Z is divided by $Z_0$, $Z_0$ being the time-of-flight of an ultrasonic pulse which has substantially not been influenced by the object to be examined and for the determination of which the same geometrical distance has been used between the transmitter and the receiver.

6. A method as claimed in Claim 3 or 4, characterized in that the short-time cross-correlation function $B(l)$ of the measurement signal (s) and of the reference signal $(s^*)$ is formed by means of the sampling values $(s_x)$ and the reference values $(s^*_y)$, the value of $l$ being determined for which the short-time cross-correlation function $B(l)$ has an absolute maximum.

7. A device for performing the method claimed in Claim 3 or 4, comprising a transmitter (2) for generating ultrasonic pulse and a receiver (3) for receiving the ultrasonic pulses, a detector device (6 ... 11) which determines the period of time expiring between the instant of transmission of a pulse and the instant a which the measurement signal generated by the receiver exceeds a predetermined value, a reconstruction unit (24) which determines the acoustic properties of the examination zone between the transmitter and the receiver from the time-of-flight of the ultrasonic pulses, and a display unit (25) for the display of the image of the examination zone determined by the reconstruction unit, characterized in that it comprises

– means (7) for the sampling of the signal received (s) at a predetermined sampling rate (l/T),

– a first memory device (16) for the storage of the sampling values $(s_x)$ thus formed,

– a second memory device (17 ... 20) in which the sampling points $(s^*_y)$ of a reference signal which corresponds to the typical variation of the measurement signal are stored with a density which is a factor K higher,

– an arithmetic unit for determining a value $l^*$ from $l$ for which the function

$$G(l) = - B(l)^2/A(l) + C(l)$$

has a minimum, subject to the susidiary condition $B(l) > 0$, or for which the function $B(l)$ has a maximum,

– and means (23) for forming a value which corresponds to the time-of-flight in accordance with the relation

$$Z = n^*T + l^*T/K - d,$$

the quantities $A(l)$, $B(l)$, $C(l)$, $l$, $K$, $n^*$, $T$ and $d$ being as defined.

Fig.1a

$K=4$
$M=7$
$N=2$
$L=K\cdot(N+1)=12$
$T=60\,ns$

Fig.1b

Fig.1c

$K=4$
$M=7$
$N=2$
$L=K\cdot(N+1)=12$
$T=60\,ns$

Fig.1d

Fig. 2

Max: $=0$;   $l^* = 0$ — 210

For $l = 1, ...., L$ — 211

$$B := \sum_{m=1}^{M} S_m \cdot S^*_{[K(m+N)+1-l]}$$ — 212

no   $B > Max$?                    yes — 213

Max: $= B$ — 214

$l^* := l$ — 215

Fig. 3